(19) Europäisches Patentamt
European Patent Office
Office européen des brevets

(11) Publication number: 0 066 001 B1

(12) **EUROPEAN PATENT SPECIFICATION**

(45) Date of publication of patent specification: **14.05.86**

(51) Int. Cl.⁴: **C 07 J 11/00,** A 61 K 31/565

(21) Application number: **81104221.7**

(22) Date of filing: **02.06.81**

(54) **Pharmaceutical compositions containing androstenes.**

(43) Date of publication of application:
**08.12.82 Bulletin 82/49**

(45) Publication of the grant of the patent:
**14.05.86 Bulletin 86/20**

(84) Designated Contracting States:
**AT BE CH DE FR GB IT LI LU NL SE**

(56) References cited:
**GB-A-1 492 746**
**US-A-3 239 542**

**JOURNAL OF MEDICINAL CHEMISTRY, vol. 6, no. 3, 6th March 1963, pages 156-161 Washington D.C., U.S.A. A. BOWERS et al.: "Steroids. CCV. Ring a modified hormone analogs. Part I. Some ring a olefins"**

**The file contains technical information submitted after the application was filed and not included in this specification**

(73) Proprietor: **Laboratoire Théramex**
**4, rue des Lilas**
**Monte-Carlo (MC)**

(72) Inventor: **Gueritee, Nicolas, Dr.**
**65 rue des Vignes**
**Paris (FR)**

(74) Representative: **Leyh, Hans, Dr,-Ing. et al**
**Patentanwälte Dr.rer.nat.Dipl.-Chem. Thomas Berendt Dr.-Ing. Hans Leyh Innere Wiener Strasse 20**
**D-8000 München 80 (DE)**

**EP 0 066 001 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention is concerned with the use of the compound 17-α-ethynyl-(5α)-2-androst-2-en-17-β-ol and its 17-β-acetate of the following formula:

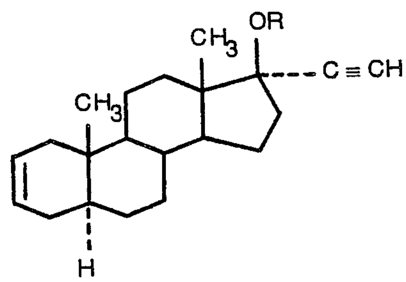

(I)

R = H

R = Ac

substantially free of a $\Delta_3$ isomer for preparing a pharmaceutical composition for a medicament for the treatment of tumor free benign breast disease, endometriosis, Redus disease in humans and pseudo gestation in animals.

A process for obtaining a substance to which they could attribute the structure and chemical formula of 17-α-ethynyl-(5-α)-androst-2-en-17-β-ol and its acetate, possessing properties that would stimulate the reticulo-endothelial system and inhibit the gonads by suppressing the pituitary gonadotrophins has been described by M. Huffman (U.S. Patent Specification 2,996,524), J.A. Edwards and A. Bowers (Chemistry and Industry, 1961, p. 1962-1963) and D. Pomonis & al. (Cancer Chemotherapy Reports, 1962, Sept., p. 31—32).

In order to be assured of the purity of the said substance, the applicant discovered that because of the process concerned this substance is not a pure product but a mixture of this product (I) with its androst-3-en isomer (II), the latter in varying but unacceptable proportions in the order of about 20%.

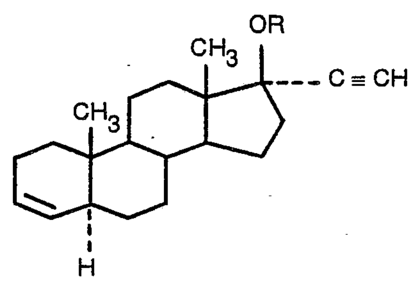

(II)

It has been found that the raw material used by the method described by Huffman in U.S. Patent Specification 2,996,524 improperly designated as 17-keto-(5α)-androst-2-en was itself a mixture of isomers delta-2 and delta-3, which are of identical polarity and thus impossible to separate by the classical methods (J. Fajkos & al., Czech.Chem.Comm., 1959, *24:* 3115—3135; P.D. Klimstra, J.Med.Chem., 1965, *8:* 45—52).

This invention is dependent on the discovery of the Sensitivity of isomer $\Delta_3$ to Jones reagent, thereby contrasting with the lack of sensitivity of isomer $\Delta_2$ to this reagent (UK Patent specification No. 1,492,746) which is a solution of chromic anhydride in acetone, in the presence of $H_2SO_4$ (Boden, K., Heilbron, L.N., Jones, E.R.H. Weedon, E., Journal Chem. Soc., 1946: 38) contrary to isomer delta-2.

When treating the raw material i.e. a mixture of (5α)-androst-2-en-17-one and (5α)-androst-3-en-17-one with Jones reagent the latter isomer is oxidised, probably into a corresponding diacid or aldehyde and thus its polarity becomes different from that of the $\Delta_2$-isomer; this enables the latter to be isolated in pure form by conventional physico-chemical means such as silica gel chromatography or other distribution techniques.

From this point, pure 17-Keto(5α), 2-androsten, after undergoing the reaction described in example No. 8 of the U.S. Patent No. 2,996,524 leads to 17β-ethynyl (5α), 2-androsten-17-β-ol in pure form, which is the product concerned in the present invention.

The reaction is suitably conducted in a suitable solvent mixture for the alkali metal salt of acetylene, such as toluene or a mixture of toluene and anhydrous t-amyl alcohol, and at a temperature of from 0°C to 50°C. The reaction is preferably carried out in an atmosphere of an inert gas e.g. nitrogen. As alkali metal salt there can also be used a complex salt, for instance the acetylide complex of lithiumethylenediamine in dimethyl sulphoxide.

The acetylation may be carried out in a conventional manner e.g. by reacting compound I in which R represents a hydrogen atom, with acetic anhydride, preferably in the presence of a catalyst, for instance para-toluenesulfonic acid.

The process for isolating pure 5α-androst-2-en-17-one from a mixture of (5α)-androst-2-en-17-one and 5α-androst-3-en-17-one comprises treating the mixture, of the two isomers with Jones reagent and isolating pure $\Delta_2$-isomer from the reaction mixture.

By this method, the (5α)-androst-3-en-17-one is converted into a compound with different physico-chemical properties, most likely to the corresponding diacid or aldehyde, and thus its physico-chemical properties, e.g. polarity, become different to that of the $\Delta_2$-isomer; this enables the latter to be isolated in pure form by conventional physico-chemical processes, e.g. crystallization from a suitable solvent or, preferably, by chromatographic separation, in particular by silica gel chromatography.

The reaction of the isomer-mixture with Jones reagent is preferably carried out in acetone solution with cooling.

The following is a method of preparation consisting in the separation of the 17-oxo-(5α)-androst-2-ene and -3-ene isomers, and in the preparation of the compound of formula I

Example

Stage 1: Separation of a mixture of 17-oxo-5α-androst-2-ene and -3-ene.

20 g of the steroid in 400 ml of acetone solution were introduced into a 1 litre flask with magentic stirring which was then cooled to −20°C and 60 ml Jones reagent were added dropwise. The flask was shaken for 1 1/4 hours, allowing it to return to room temperature. 100 cc methanol were then added and the mixture shaken for 15 minutes. The solvents were distilled off and the residue taken up in 700 ml water. The precipitate was filtered, washed with water and dissolved in 500 ml methylene chloride. The organic solution was then dried with sodium sulphate and distilled. The residue (18,5 g) was purified by silica gel column chromatography. Elution with benzene produced 11 g of pure $\Delta_2$-isomer. The NMR spectrum ($CDCL_3$, TMS) of this derivative has olefinic protons at $C_2$ and $C_3$ as a broad signal at 334 Hz (W/2:5 Hz), while the N.M.R. spectrum of the $\Delta_3$-isomer has 4 broad olefinic proton signals between 312 and 348 Hz.

Stage 2: Preparation of 17α-ethynyl-(5α)-androst-2-en-17β-ol free of DELTA-3.

1.2 g of potassium was dissolved in 30.5 ml of anhydrous t-amyl alcohol. A solution of 1.1 g of pure 5α-androst-2-en-17-one, obtained as in Stage 1, in 40 ml of anhydrous toluene was added and nitrogen was passed through the mixture to dispel air. The resulting solution was stirred for 15 hours while a slow stream of purified anhydrous acetylene was bubbled through. At the end of this period, 300 ml of ice water were added and the pH adjusted to 1 with 50% aqueous hydrochloric acid. The resulting solution was distilled to remove all volatile organic materials. It was then cooled to 0°C, extracted with ether, the ether extract was then washed with water, dried with sodium sulphate and the solvent evaporated. The residue of 17α-ethynyl-(5α)-androst-2-en-17β-ol was recrystallized from hexane.

Stage 3: Preparation of the 17-acetate of 17α-ethynyl-(5α)-androst-2-en-17β-ol.

600 mg of para-toluenesulphonic acid were introduced into a solution of 600 mg of 17α-ethynyl-(5-α)-androst-2-en-17β-ol in 10 ml acetic anhydride. The solution thus obtained was allowed to stand at 20°C for 18 hours, protected from air. It was then poured into 100 ml of ice water and the mixture cooled for 3 hours. The precipitate of 17α-ethynyl-5α-androst-2-en-17β-acetate was collected by filtering; it was washed with water, then dried and recrystallized from methanol.

The product obtained in Stage 2 was in the form of white crystals which melted at 160°—162°C (Kofler). These properties may be compared with those of the mixture obtained by the process described by the above-named authors: according to the proportion of isomer "DELTA—3", the melting point of the mixture (determined under the same conditions) varies, approaching the value 198°—199°C (Kofler) for pure "DELTA—3". Likewise, the acetate obtained in Stage 3 above, was in the form of white plates and melted at 130—131°C (Kofler).

The compounds of the present invention are useful in unexpected therapeutic fields that are different from and even contrary to those referred to in the above named literature.

It has been known that "DELTA—2", contrary to what was stated in previous literature, which concerns the mixture of the 2 isomers: $\Delta_2$ and $\Delta_3$ (thereafter said "MIX"), stimulates the gonads and therefore possess under certain conditions by different routes, a clomiphene-like stimulating effect on the pituitary gonadotrophins.

The two compounds: pure 17α-ethynyl-(5α)-androst-2-en-17β-ol and its acetate and especially the acetate, are useful in the following therapeutic applications (this list is not limiting):

1) Pituitary-gonadal dysfunction in both sexes, including some forms of infertility, at a daily dose of 25 to 100 mg;

2) Endometriosis, at a daily dose of 100 to 300 mg;

This field of therapeutic application of the two compounds is particularly useful and unexpected one.

The condition is characterized, amongst others by intense pain towards the end of the menstrual cycle, due to proliferation under the effect of physiologic estrogens, of fragments of endometrial tissue disseminated outside the uterine cavity.

Treatment of this condition not infrequently requires surgical removal or drug suppression of the ovarian function.

The clinical results obtained by "DELTA—2" acetate in a group of patients are summarised hereafter.

Thirty-one patients, from 18 to 41 years of age, suffering from evolutive vaginal, ovarian, peritoneal and/or uterine endometriosis, were treated from 2 to 9 months with compound "DELTA—2" at a daily dose of 100 mgs.

The condition, including topography of the lesions, was estimated, before and after treatment, mainly by anatomic and pathologic criteria:

— inspection (if vaginal localisation) or
— palpation, or
— laparoscopy, or
— laparotomy, or
— sonography, or
— hysterosalpingogram, and or
— sampling of biopsies for cytologic and histologic examination.

The usefullness of the treatment was also appreciated by the disappearance of the pelvic pain which is the principal subjective symptom of the disease.

The overall results can be summarized as follows:

a) *Functional signs;* they
— were present in 24 cases out of 31;
— had completely disappeared in 18 cases during the 1st month of the treatment;
— had very improved in 3 cases;
— had persisted in 3 cases;

b) *Anatomic evolution of the lesions*
Anatomic control was made in 27 cases.
Endometriosis had
— completely disappeared in 21 cases;
— resisted in 1 case;

c) *Menstrual cycles*
Treatment provoked
— total amenorrhoea up the 1st month, in 16 cases; the 2nd month, in 1 case;
— amenorrhoea with spotting in 2 other cases;
— irregular bleedings in 5 cases;
— menses persisted, regular, in 5 cases.

Menses occurred during the 1st month following the arrest of treatment, in 17 cases out of 19 cases of amenorrhoea.

3) Certain conditions involving bone atrophy(osteoporosis), osteolysis (bone metastases of various cancers) Paget's bone disease, retardation in the bone repair process (delay in consolidation of fractures of grafts; pseudarthrosis) associated or not with hyperparathyroidism at a daily dose of 10 to 100 mg;

4) *Treatment of Pseudo-gestation in animals with DELTA—2*

Clinically, pseudo-gestation is characterized by more or less serious troubles which appear 2 months after estrus. The symptoms and the anatomical lesions are variable in intensity and duration. There is no evidence of any inflammatory state.

The ovaries show several enlarged corpus luteum, uterus is thickened, hyperplasic and filled with white serosal liquid. There is no way to predict the occurrence of the syndromes and, in particular, there is no specific hormonal modification that could explain the phenomenon. Recently, a few authors have outlined the possible role of prolactin, and therefore, treatment with bromocriptin was tried with little success. In fact, the mechanism of pseudogestation is not well known and all treatment tried to date met with very little success. The table hereafter summarizes the results obtained.

Nine bitches were treated for 10 days and the daily dosage of DELTA—2 compound ranged from 0.5 mg/kg to 1.5 mg/kg/day.

In the 2 failures that have been observed the daily dosage of DELTA—2 was low, i.e., less than 1 mg/kg.

The treatment has been well tolerated as opposed to other treatments previously used (androgens and estrogens).

The high rate of therapeutic success and the rapidity of clinical symptoms disappearance show that DELTA—2 compound is a safe, effective and well tolerated treatment of the pseudogestational syndrome of the bitch. There were no particular side effects.

TABLE

| No. Cases | Breed | Years | Dose unit/ day | Dose mg/kg | Results |
|---|---|---|---|---|---|
| 1 | Artesien normand | 3.5 | 3 | 0.75 | – |
| 2 | Epagneul breton | 1 | 3 | 1 | + |
| 3 | Fox | 1 | 1 | 1 | ++ |
| 4 | Fox | 1.5 | 1 | 1 | +++ |
| 5 | Fox | 4 | 1 | 1 | +++ |
| 6 | Chowchow | 3 | 3 | 0.7 | +++ |
| 7 | Fox | 4 | 2 | 1 | +++ |
| 8 | Groenendael | 3 | 4 | 0.8 | ++ |
| 9 | Fox | 10 | 1 | 0.6 | – |

– no improvement after 10 days
\+ improvement but no disappearance of symptoms after 10 days
++ disappearance of symptoms within 10 days.
+++ disappearance of symptoms within 7 days.

5) *Treatment of Human Benign Breast Disease*

A. *The Disease*

Benign breast disease is frequently described as a broad entity encompassing several pathological changes and clinical findings.

The disease is most frequently diagnosed clinically on the basis of breast pain or tenderness and on fibrocystic changes which, on palpation of the breast, characteristically feel diffuse nodular, granular or lumpy. The aetiology is not clearly defined.

Estrogens and progestogens, combined and sequential, and androgens have been used with varying degrees of success and also analgesics and diuretics.

Five patients presenting with benign breast disease were treated. Among these 5 patients:

— 3 had fibro-cystic nodular changes of the breast accompanied by breast pain and tenderness typically corresponding to Reclus disease.

— the 2 others showed diffuse lumpy changes of the breast with pain and tenderness.

These 5 patients were treated with DELTA—2 compound at a daily dose of 100 mg for 9 months. Overall, the treatment was well tolerated. There were no headaches, no modification of arterial tension, and no weight gain throughout the treatment. All the patients became amenorrheic within the first month of treatment and this was accompanied by a diminution of plasma estrogen and progesterone level and a disappearance of the ovulatory pic of FSH and LH. The patients remained amenorrheic throughout the treatment.

Clinical symptomatology of the benign breast disease, i.e., pain tendernness and physical changes disappeared within 3 months of the onset of treatment. All the patients remained symptom free as to their breast disease during the therapeutic period and after cessation of treatment.

The disappearance of the clinical symptoms of the disease was correlated with a normalization of the thermographic data. After cessation of treatment all the patients resumed normal menstrual cycles.

In conclusion, DELTA—2 compound at daily dosage of 100 mg is a safe, effective, and well tolerated treatment of benign breast disease. The side-effects of this treatment, i.e., amenorrhoea due to hypophysal inhibition, should limit its use to severe forms of benign breast disease.

Compound I (acetate) as embodied by this invention, is essentially, but not exclusively, destined for oral administration, in one of the usual pharmaceutical forms: tablets, capsules, pills or else in a liquid vehicle, as a suspension or a solution in olive oil, in gelatine capsules, or in a syrup or a linctus.

Taking the above precise dosage limits into account according to the indications, the concentrations per unit dose should preferably be: 2,5 mg, 5 mg, 10 mg, 25 mg, 100 mg.

This compound can also be administered in the form of suppositories containing 25 or 50 mg, for rectal or vaginal insertion, or in the form of a lotion of spray containing 10 mg/ml in an appropriate vehicle.

Lastly, this compound can be used in an injectable form, for intra-muscular administration, at a concentration of 25 to 50 mg per ampoule, in an acceptable lipid solvent (olive or sesame oil, benzyl alcohol etc. . . .).

**Ciaim**

Use of a compound of the formula

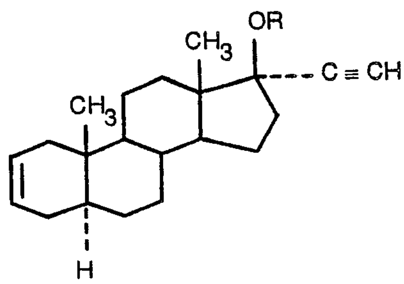

wherein R represents a hydrogen atom or an acetyl group substantially free of a $\Delta_3$ isomer for preparing a pharmaceutical composition for a medicament for the treatment of tumor free benign breast disease, endometriosis, Reclus disease in humans and pseudo gestation in animals.

**Patentanspruch**

Verwendung einer Verbindung der Formel

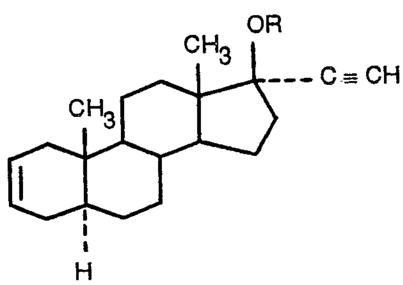

in der R ein Wasserstoffatom oder eine Acetylgruppe bedeutet und die praktisch kein $\Delta$ 3-Isomer enthält, zur Herstellung einer pharmazeutischen Zubereitung für ein Arzneimittel zur Behandlung der tumorfreien gutartigen Brustdrüsenkrankeit, Endometriosis und Reclus' Krankheit beim Menschen und der Scheinträchtigkeit bei Tieren.

**Revendication**

Utilisation d'un composé de structure chimique:

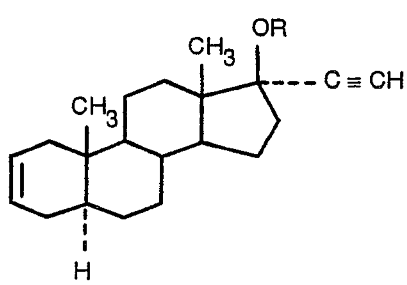

où R représente un atome d'hydrogène ou un radical acètyl, substantiellement dépourvu de l'isomère $\Delta$ 3, pour préparer une composition pharmaceutique destinée au traitement, dans l'espèce humaine des: mastopathies bénignes non tumorales, endométriose et maladie de Reclus et au traitement de la pseudogestation chez les animaux.